# EUROPEAN PATENT SPECIFICATION

(11) **EP 1 393 733 B1**
(45) Date of publication and mention of the grant of the patent: **23.08.2006**
(21) Application number: 03015616.0
(22) Date of filing: 16.07.2003
(51) Int. Cl.: A61K 31/352, A61K 36/00, A61P 17/04

(54) **Use of flavonoid-derivatives for the treatment of atopic eczema**
Verwendung von Flavonoid-Derivaten zur Behandlung von atopischem Ekzem
Utilisation de dérivés de flavonoides pour le traitement d'eczéma atopique

(30) Priority: 02.09.2002 DE 10240923
(43) Date of publication of application: 03.03.2004
(73) Proprietor: Merck Patent GmbH, 64293 Darmstadt (DE)
(72) Inventor: Buchholz, Herwig Dr., 60599 Frankfurt (DE); Wirth, Corinna Dr., 64283 Darmstadt (DE)

(56) References cited:
- US-A1- 2001 031 735
- US-B1- 6 399 112
- KOMBAL R ET AL: "Flavan-3-ols and flavonoids from Potentilla anserina" PLANTA MEDICA, vol. 61, no. 5, 1995, pages 484-485, XP008025193 ISSN: 0032-0943
- ISHIGURO K ET AL: "ANTIPRURITIC EFFECT OF FLAVONOL AND 1,4-NAPHTHOQUINONE DERIVATIVES FROM IMPATIENS BALSAMINA L" PHYTOTHERAPY RESEARCH, JOHN WILEY & SONS LTD. CHICHESTER, GB, vol. 11, 1997, pages 343-347, XP002929083 ISSN: 0951-418X
- HARBORNE J B ET AL: "Advances in flavonoid research since 1992" PHYTOCHEMISTRY, PERGAMON PRESS, GB, vol. 55, no. 6, November 2000 (2000-11), pages 481-504, XP004291674 ISSN: 0031-9422
- DELLA LOGGIA R ET AL: "TOPICAL ANTI-INFLAMMATORY ACTIVITY OF SOME FLAVONOIDS FROM QUERCUS-ILEX LEAVES" PLANTA MEDICA, vol. 55, no. 1, 1989, pages 109-110, XP008025194 36TH ANNUAL CONGRESS OF THE SOCIETY FOR MEDICINAL PLANT RESEARCH, FREIBURG, WEST GERMANY, SEPTEMBER ISSN: 0032-0943
- TUBARO A ET AL: "TOPICAL ANTI-INFLAMMATORY ACTIVITY OF A NEW ACYLATED FLAVONOID" AGENTS AND ACTIONS, BIRKHAEUSER VERLAG, BASEL, CH, vol. 26, no. 1/2, 1989, pages 229-230, XP008001961 ISSN: 0065-4299
- PATENT ABSTRACTS OF JAPAN vol. 018, no. 371 (C-1224), 13 July 1994 (1994-07-13) & JP 06 100584 A (JUMOKU CHUSHUTSU SEIBUN RYIO GIJUTSU KEN), 12 April 1994 (1994-04-12)
- RECIO M C ET AL: "Studies on the radical scavenger and anti-inflammatory activities of tiliroside" INFLAMMATION RESEARCH, vol. 50, no. Supplement 3, September 2001 (2001-09), page S205 XP008025202 5th World Congress on Inflammation;Edinburgh, Scotland; September 22-26, 2001 ISSN: 1023-3830
- SALA ARACELI ET AL: "Anti-inflammatory and antioxidant properties of Helichrysum italicum" JOURNAL OF PHARMACY AND PHARMACOLOGY, vol. 54, no. 3, March 2002 (2002-03), pages 365-371, XP008025203 ISSN: 0022-3573
- HEALTH TECHNOLOGY ASSESSMENT, vol. 4, no. 37, 2000, pages 1-4,
- YMASHITA H. ET AL: 'Dermal mast cells play a central role in the incidence of scratching behavior in mice induce by multiple application of the hapten, 2,4,6-trinitrochlorobenzene' EXPERIMENTAL DERMATOLOGY vol. 14, 2005, pages 438 - 444
- MORGAN D.W.; MARSHALL L.A.: 'In vivo models of inflammation', 1999, BIRKHÄUSER VERLAG, BASEL * page 179 - page 180 *

## Description

The invention relates to the use of certain flavonoid derivatives for the preparation of formulations which are suitable for the prophylaxis and/or therapy of atopic eczema, and to formulations which comprise flavonoid derivatives of this type.

According to http://yavivo.lifeline.de (BertelsmannSpringer Medizin Online GmbH), atopic eczema is a common disease in industrialised countries. Studies have shown that on any particular day about 2.5% of the total population of Europe is suffering from itchy skin changes caused by atopic eczema.

According to ROCHE Lexikon Medizin [ROCHE Lexicon of Medicine], 3rd Edition, eczema is an acute, sub-acute or chronic disease of the epidermis with extensive [lacuna] which are not clearly delimited from healthy skin, for example as nodulation, vesiculation and flaking with initial and accompanying skin reddening (erythema), possibly with hornification (eczema callosum), and also with participation of deeper skin layers (the term eczema cannot be clearly delimited from the term dermatitis and is frequently replaced by the latter in Anglo-Saxon language regions). A raised immune response, abrasions and damage to the skin and its protective sheaths ("barriers") - based on sweat and sebaceous gland activity - and infestation with pathogens (bacteria, fungi) can have a promoting or initiating action; special forms are endogenous or constitutional eczema (eczema atopicum), microbial eczema (due to bacteria and fungi as eczematogens) and seborrhoeic eczema (eczema seborrhoicum).

Atopic eczema (eczema atopicum; endogenous eczema, essential eczema, dermatitis atopica) is eczema as a consequence of constitutional hypersensitivity (atopy), where it is frequently not possible to observe a particular eczematogen. The skin manifestations of "atopic diathesis" are age-dependent with respect to reaction site and type: in infants as facial eczema (milk crust, infantile eczema), in schoolchildren and adults as neurodermatitis, in adults also with rather scattered foci with papules (prurigo) on the trunk and limbs; also very discreet forms, for example dermatitis sicca; often accompanied by asthma or rhinoconjunctivitis.

Inflammatory skin changes of this type are generally treated with a mild or moderate glucocorticoid preparation in order to counter the formation of chronic skin changes. However, sudden discontinuation of the glucocorticoid can result in problems since this can favour the inflammation reaction flaring up again. Furthermore, glucocorticoid-containing ointments and creams can only be employed in the short term since the skin can become thinner on application over a number of weeks.

Owing to these disadvantages, there is a demand for alternative or complementary active ingredients for the prophylaxis and/or therapy of eczema, in particular atopic eczema.

The object of the present invention was therefore to provide suitable active ingredients.

The earlier international patent application PCT/EP 02/01200 describes compounds of the formula I 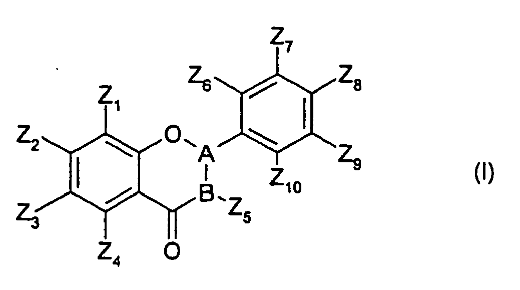 in which
- Z₁ to Z₄ and Z₆ to Z₁₀: are each, independently of one another, H, OH, CH₃COO, alkoxy, hydroxyalkoxy, mono- or oligoglycoside radicals and where the alkoxy and hydroxyalkoxy groups may be branched or unbranched and can have from 1 to 18 carbon atoms, 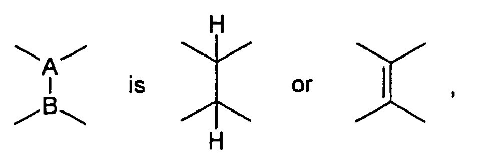
- Z₅: is a mono- or oligoglycoside radical, where at least one radical selected from 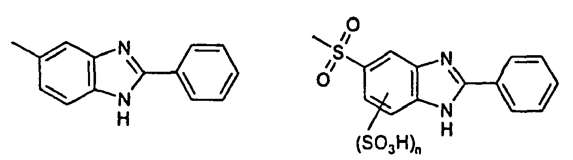 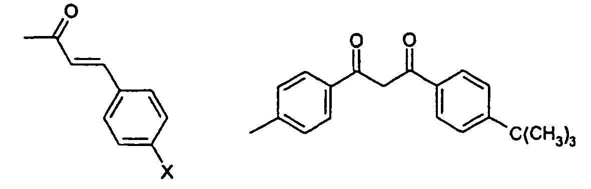 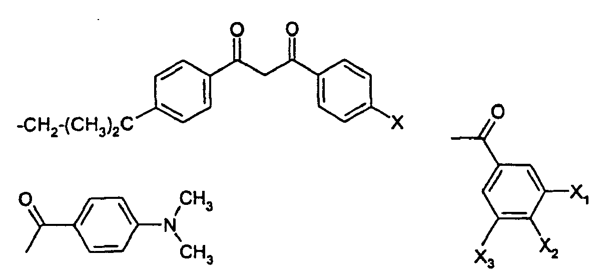 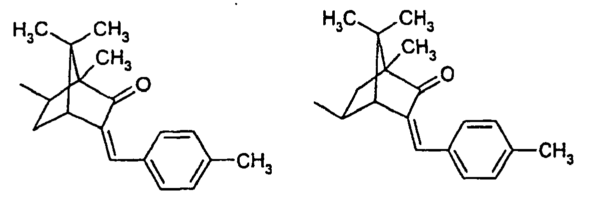 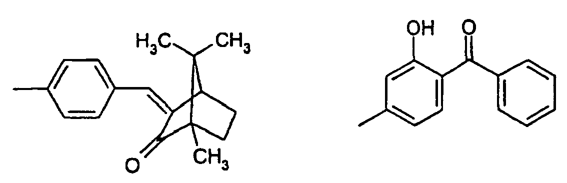 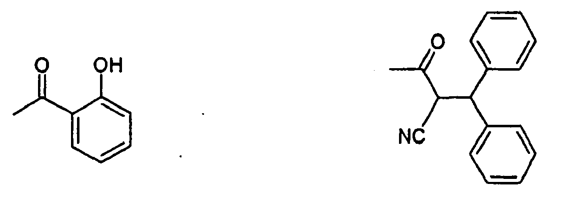 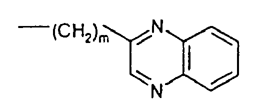 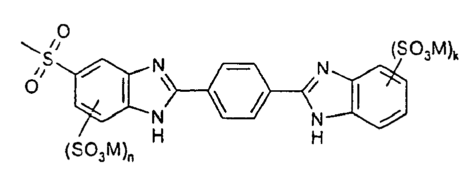 in which X, X₁, X₂ and X₃ are each, independently of one another, OH, CH₃COO, an alkoxy radical having from 1 to 8 carbon atoms or a monoglycoside radical, n is 0, 1, 2 or 3, m is 0 or 1, k is 0, 1, 2, 3 or 4, and M is H, Na or K,
is bonded to this glycoside radical, in each case via an -O- group, and in which one or more hydrogen atoms in the OH groups of the glycoside radicals mentioned in the substituents Z₁ to Z₁₀ may each, independently of one another, also be replaced by acetyl or by alkyl radicals having from 1 to 8 carbon atoms, and where, in each case independently of one another, sulfate or phosphate may also be bonded to one or more hydroxyl groups of the radicals mentioned in the substituents Z₁ to Z₁₀. These compounds are suitable for use in cosmetic and pharmaceutical formulations. In particular, PCT/EP 02/01200 describes the suitability of these compounds for use as UV filters and as active ingredient for protection against oxidative stress and for preventing skin ageing. It is furthermore described that these compounds exhibit antiallergic, antiinflammatory, inflammation-inhibiting and antiirritative properties and can thus be used for the treatment or preventive treatment of allergies, inflammation and irritation, in particular of the skin.

Surprisingly, it has now been found that these compounds are eminently suitable for the treatment of eczema.

A first subject-matter of the present invention is therefore the use of compounds of the formula IA described here below or salts thereof for the preparation of a formulation for the prophylaxis and/or therapy of eczema.

For the purposes of the present invention, the term formulation here is taken to mean a cosmetic, dermatological or pharmaceutical formulation which is suitable for topical application. Typical formulations comprise conventional excipients which are tolerated by the skin and have been tested in accordance with the intended use and optionally further adjuvants and active ingredients.

A further subject-matter of the present application is a formulation for topical application comprising
a) at least one compound of the formula IA as described here below,
b) a skin-tolerated excipient,
c) optionally one or more further active ingredients having a skin-care and/or inflammation-inhibiting action.

Preferred formulations comprise at least one inflammation-inhibiting active ingredient c), which is preferably selected from the glucocorticoids or tacrolimus.

Tacrolimus has been isolated from the fungus Streptomyces tsukukaensis and exhibits an immunosuppressive action.

Suitable glucocorticoids are, for example, prednisone, cloprednol, triamcinolone, methylprednisolone, dexamethasone, betamethasone, desoximetasone, clobetasone butyrate, halcinonide, clobetasol propionate, prednisolone, hydrocortisone butyrate, betamethasone dipropionate, fluocinolone acetonide, betamethasone valerate, hydrocortisone (cortisol), cortisone acetate, prednicarbate, diflucortolone valerate, triamcinolone acetonide, fluocortolone and fluocortolone 21-hexanoate.

Formulations of this type which comprise an active-ingredient combination of at least one compound of the formula IA and at least one of the above-mentioned further active ingredients exhibit a particularly strong inflammation-inhibiting action.

In particular, it has been found that the compounds of the formula IA and the formulations according to the invention can be employed particularly advantageously in the treatment of atopic eczema, such as, in particular, milk crust, neurodermatitis, prurigo and dermatitis sicca.

It has been found here that the compounds of the formula IA
- are able greatly to reduce the acute symptoms,
- are able to reduce the frequency of occurrence of acute symptoms,
- in general contribute to an improvement in the skin picture.

Known compounds of the formula IA are, for example, kaempferol 3-(6"-galloylglucoside) and kaempferol 3-(6"-p-coumarylglucoside), which is also known as tiliroside.

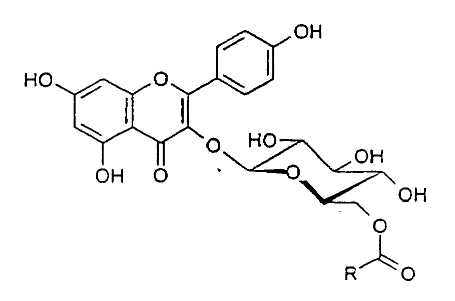

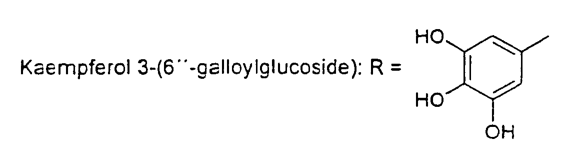

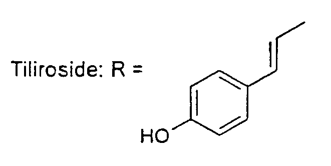

DE 195 44 905 A1 describes, for example, a process for the preparation of plant extracts containing tiliroside and the use of the plant extracts in medicaments and food products.

DE 199 22 287 A1 describes tiliroside as a starting flavonoid for the preparation of tiliroside esters whose acid unit contains from 3 to 30 carbon atoms. These esters are used in cosmetics. However, DE 199 22 287 A1 does not describe any formulations comprising tiliroside.

Furthermore, compounds of the formula IA, such as, for example, tiliroside, have only a weak inherent colour. The weak inherent colour is, for example, a major advantage if an inherent colour of the ingredients is undesired in the products for aesthetic reasons.

In a preferred embodiment, the compounds present in the formulations according to the invention are selected from the compounds of the formula IA 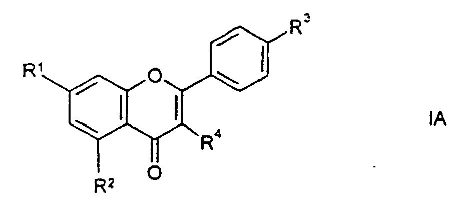 in which
- R¹, R² and R³: are each, independently of one another, OH, CH₃COO, an alkoxy radical having from 1 to 8 carbon atoms or a monoglycoside radical,
- R⁴: is a mono- or diglycoside radical, where at least one group selected from 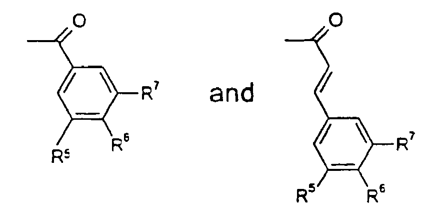 where R⁵, R⁶ and R⁷ may each, independently of one another, be H or have the meaning of the radicals R¹ to R³, is bonded to the glycoside radical, in each case via an -O-group, and in which one or more hydrogen atoms in the OH groups of the glycoside radical(s) may each, independently of one another, also be replaced by acetyl or by alkyl radicals having from 1 to 8 carbon atoms, and where, in each case independently of one another, sulfate or phosphate may also be bonded to one or more hydroxyl groups of the compounds of the formula IA.

In a preferred embodiment, the radical R² in the compounds of the formula IA is selected from OH, CH₃COO and an alkoxy radical having from 1 to 8 carbon atoms.

In the compounds of the formula IA, all OH groups of the mono- or diglycoside radical of R⁴ may be esterified with a group of the formula

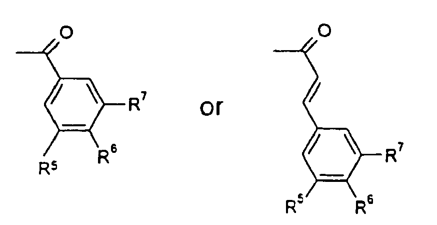

Preferably, however, only one or two of the radicals derived from these radicals is (are) bonded to the glycoside radical.

If R⁴ is a mono- or diglycoside radical in which one or more hydrogen atoms of the OH groups have been replaced by acetyl or alkyl radicals, all OH groups for which replacement is possible have then preferably been replaced by acetyl or alkyl.

Of the alkoxy radicals having from 1 to 8 carbon atoms mentioned in the compounds of the formula IA, the methoxy group is preferred. Of the alkyl radicals having from 1 to 8 carbon atoms mentioned in the compounds of the formula IA, the methyl group is preferred.

The mono- and diglycoside radicals mentioned in the compounds of the formula IA are preferably built up from glucose units.

Preferred compounds IA1 to IA13 selected from the compounds of the formula IA are indicated below:

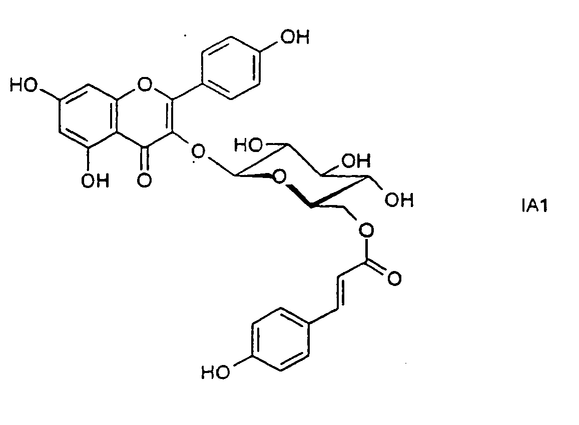

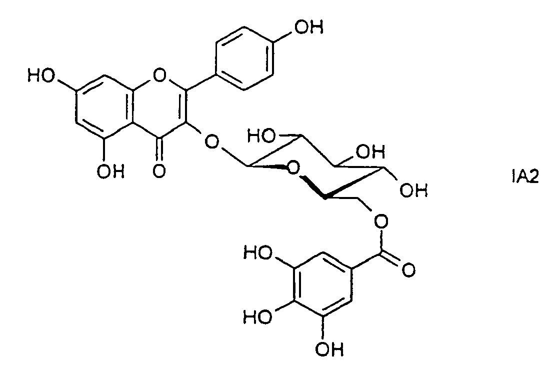

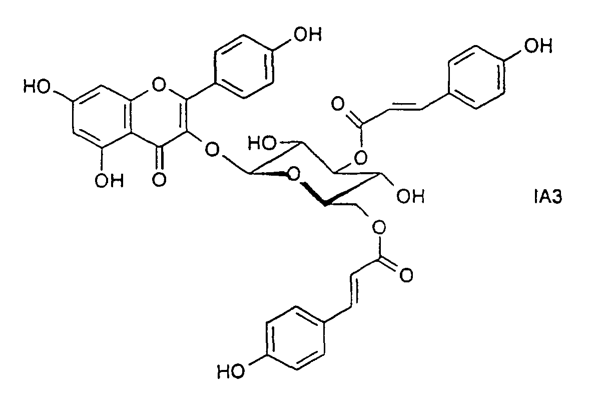

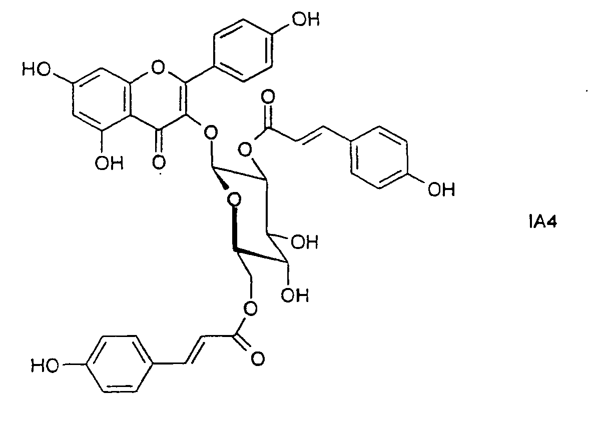

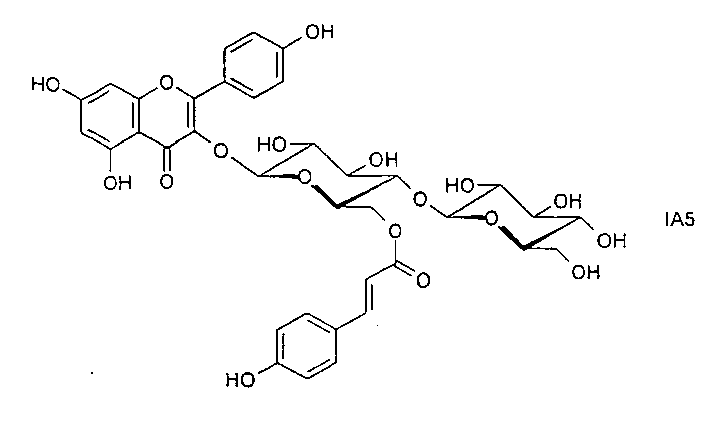

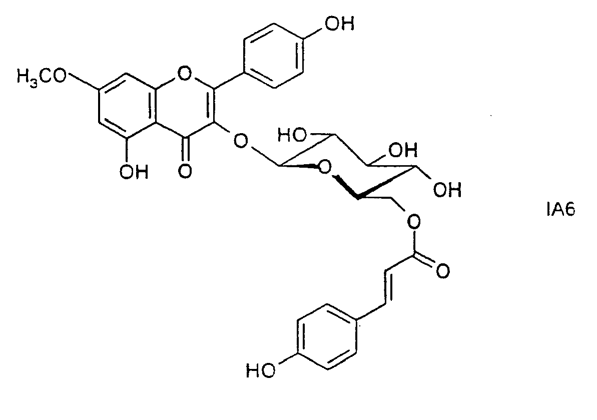

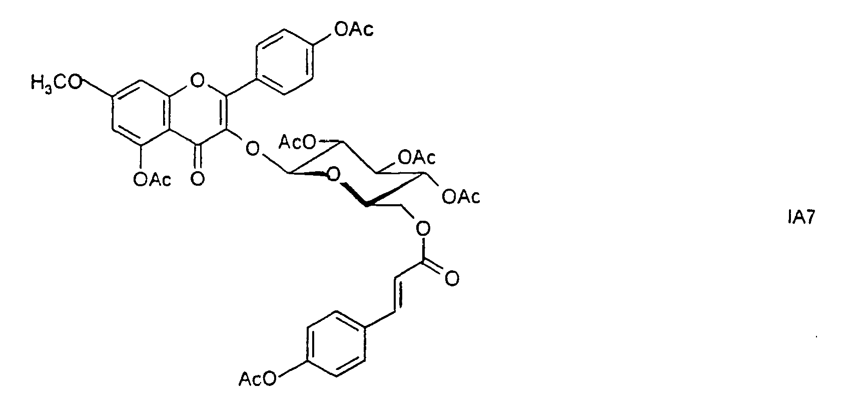

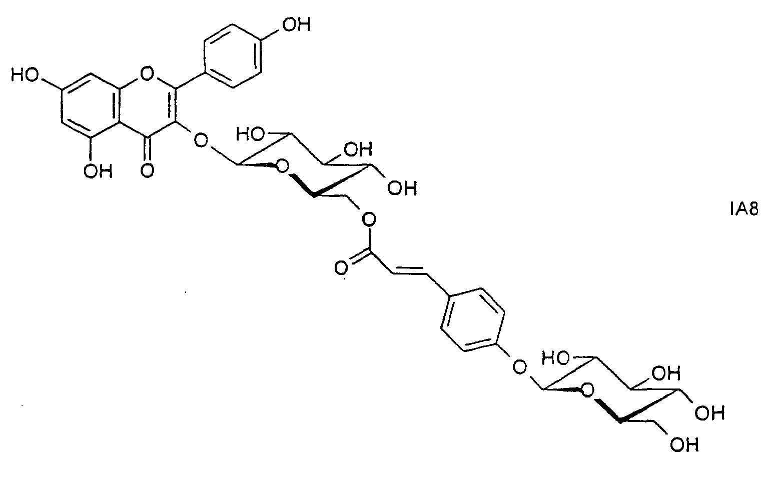

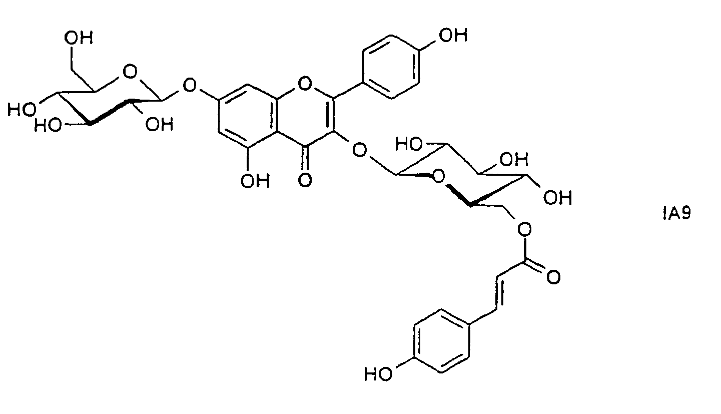

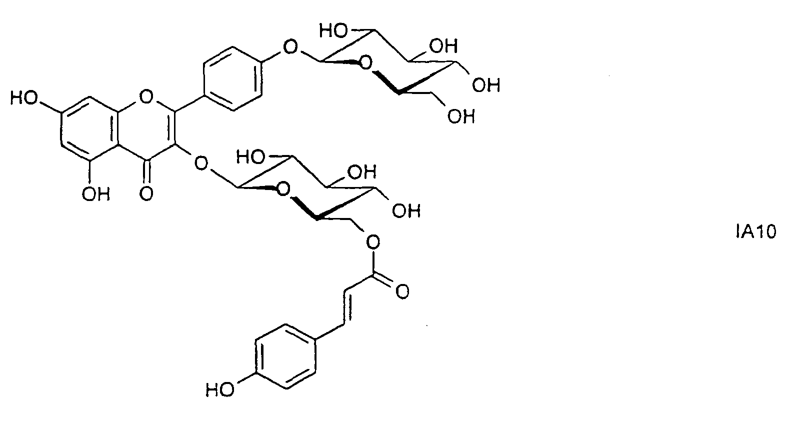

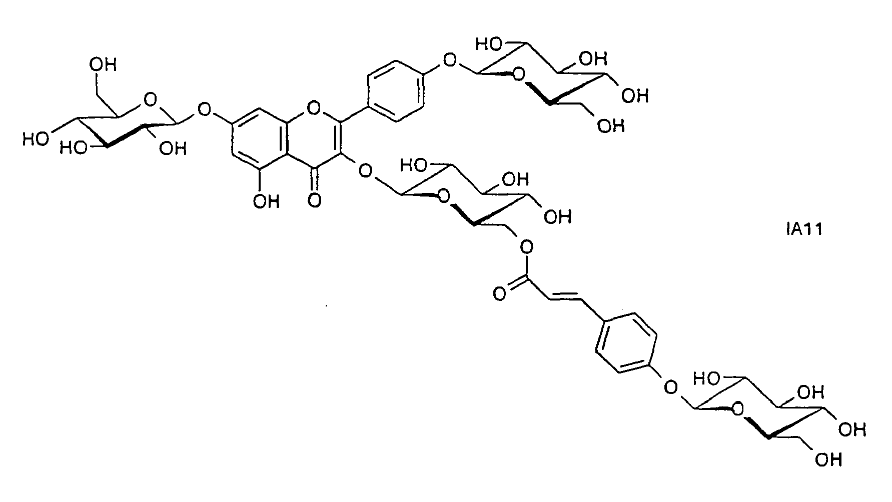

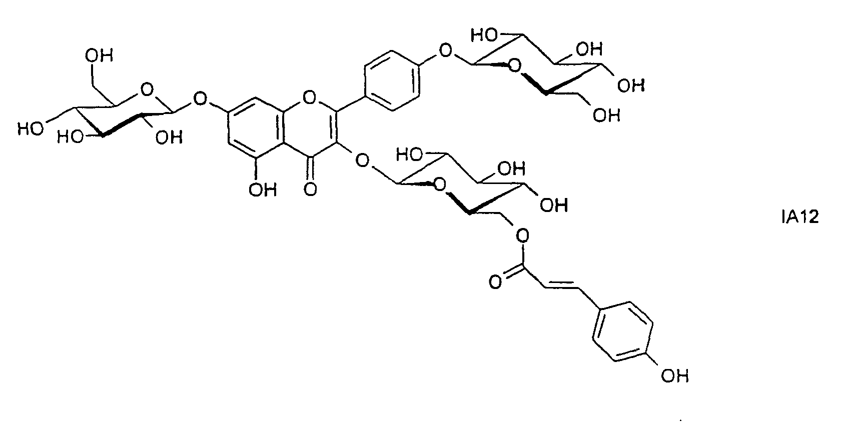

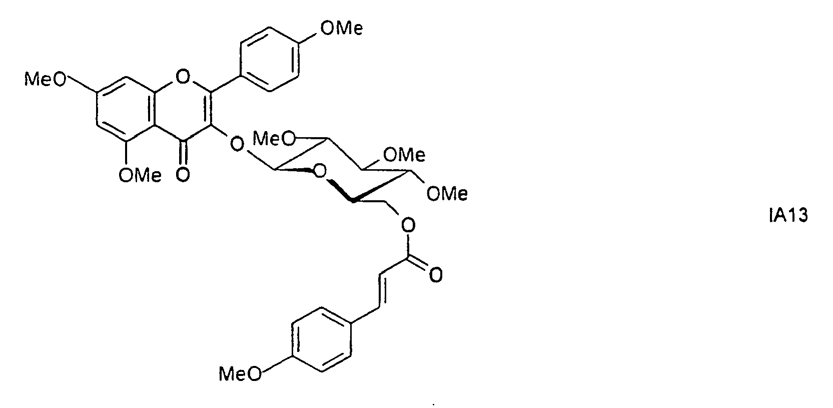

In the compounds of the formulae IA1 to IA13 mentioned above, Me is methyl and Ac is acetyl.

Of the compounds of the formula IA, particular preference is given to the compounds of the formulae IA1 and IA2. Very especial preference is given to the compound of the formula IA1, i.e. tiliroside.

In a further preferred embodiment, the compounds of the formula IA present in the formulations according to the invention are selected from the compounds in which
- R¹, R² and R³: are each, independently of one another, OH, an alkoxy radical having from 1 to 8 carbon atoms or a monoglycoside radical,
- R⁴: is a mono- or diglycoside radical, where at least one group selected from 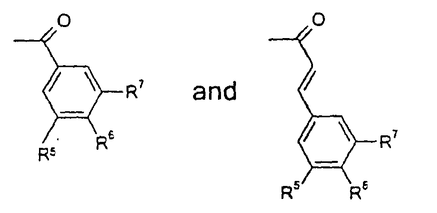 where R⁵, R⁶ and R⁷ are each, independently of one another, H, OH, an alkoxy radical having from 1 to 8 carbon atoms or a monoglycoside radical, is bonded to the glycoside radical, in each case via an -O- group, and
in which one or more hydrogen atoms in the OH groups of the glycoside radical(s) may each, independently of one another, also be replaced by alkyl radicals having from 1 to 8 carbon atoms, and where, in each case independently of one another, sulfate or phosphate may also be bonded to one or more hydroxyl groups of the compounds of the formula IA.

In these compounds of the formula IA, R¹ to R³ are preferably each, independently of one another, OH or an alkoxy radical having from 1 to 8 carbon atoms.

Some compounds of the present invention such as, for example, tiliroside, can be isolated from plants, for example from plants of the genera Althaea, Aristolochia, Helianthemum, Lindera, Magnolia, Platanus, Potentilla, Quercus, Rosa, Sida, Sorbus and/or Tilia. These compounds can be processed further either in isolated form or in non-isolated form, i.e., for example, incorporated into formulations in the form of an extract or in the form of a purified extract or alternatively in the form of the pure substance prepared from the plant extract. Of the said genera, the following species are preferred: Althaea officinalis, Althaea rosea, Aristolochia heterophylla, Helianthemum glomeratum, Lindera megaphylla, Magnolia salicifolia, Platanus acerifolia, Platanus occidentalis, Potentilla anserina, Quercus pubescens, Quercus suber, Quercus laurifolia, Quercus ilex, Ouercus imbricaria, Quercus virginiana, Rosa pomifera, Sida rhombifolia, Sida poeppigiana, Sida cordifolia, Sida glaziovii, Sorbus pendula, Tilia argenta and Tilia cordata.

If the formulation according to the invention comprises tiliroside, this compound is, in a further preferred embodiment, has been used for the preparation of the formulation in the form of a plant extract, a purified plant extract or in the form of the pure substance prepared from the plant extract. In formulations of this type, the plant extract comprises, for example, from 1 to 100% by weight of tiliroside. In one embodiment, the plant extract preferably comprises from 5 to 90% by weight of tiliroside. In a further embodiment, the plant extract preferably comprises from 30 to 100% by weight, particularly preferably from 60 to 100% by weight and especially preferably from 90 to 100% by weight of tiliroside. In a further preferred embodiment, the plant extract has been isolated by extraction from the Sida glaziovii plant.

In all uses according to the invention in which tiliroside is used, tiliroside can be used, for example, in the form of a synthetically prepared substance, in the form of a plant extract, a purified plant extract or an individual substance or in the form of a pure substance isolated from the plant extract. In a preferred embodiment, tiliroside is used in the form of a plant extract, a purified plant extract or in the form of the pure substance prepared from the plant extract.

The compounds of the present invention can be isolated or prepared by methods which are well known to the person skilled in the art and are described in the literature (for example in the standard works, such as Houben-Weyl, Methoden der organischen Chemie [Methods of Organic Chemistry], Georg-Thieme-Verlag, Stuttgart).

For example, tiliroside occurs in plants and can be isolated by extraction. The plant extracts are prepared by conventional methods of extraction of the plants or plant parts. Suitable extraction methods may be: maceration, remaceration, digestion, agitation maceration, fluidised-bed extraction, ultrasound extraction, countercurrent extraction, percolation, repercolation, evacolation, diacolation or solid/liquid extraction with continuous reflux, which is carried out in a Soxhlet extractor.

The solvent used for the extraction can be, for example, water or an alcohol. It can be ascribed to the general knowledge of the person skilled in the art how these extractions can be carried out in detail and the resultant crude extracts can be purified by generally conventional methods.

One possible synthetic route for tiliroside is, for example, also described in B. Vermes, H. Wagner, Stud. Org. Chem. (Amsterdam) (1982), Volume date 1981, 11 (Flavonoids, Bioflavonoids), 161-167 and in B. Vermes, V.M. Chari, H. Wagner, Helv. Chim. Acta (1981), 64(4), 1964-1967.

The synthesis of tiliroside is shown in scheme 1. 4',7-Dibenzylkaempferol (**1**) [H. Wagner, H. Danninger, O. Seligmann, M. Nógrádi, L. Farkas, N. Farnsworth, Chem. Ber. 103 (1978) 3768] is reacted with 2,3,4-tri-O-acetyl-6-O-chloroacetyl-β-D-glucopyranosyl bromide (**2**) in the presence of Ag₂CO₃ and pyridine to give compound **3.** Compound **2** can be prepared by the method described in D.Y. Gagniere, P.J.A. Wottero, Carbohydrate Res. 28 (1973) 1965. Catalytic debenzylation and subsequent careful acetylation of compound **3** gives compound **4**, from which compound **5** can be obtained after removal of the chloroacetyl group using thiourea. In this compound, only one hydroxyl group is free, meaning that the esterification of compound **5** can proceed selectively. The esterification using the acid chloride p-acetylcoumaroyl chloride **6** can be carried out in a mixture of pyridine and dichloromethane. An excess of acid chloride and a long reaction time (about 96 hours) at room temperature are necessary to ensure that the esterification proceeds to completion. The final step, the selective saponification of the 7 acetyl groups in compound **7**, can be carried out by the method described in G. Zemplén, Chem. Ber. 59 (1926) 1258. This is carried out using a catalytic amount of NaOCH₃ and a calculated amount of methanol.

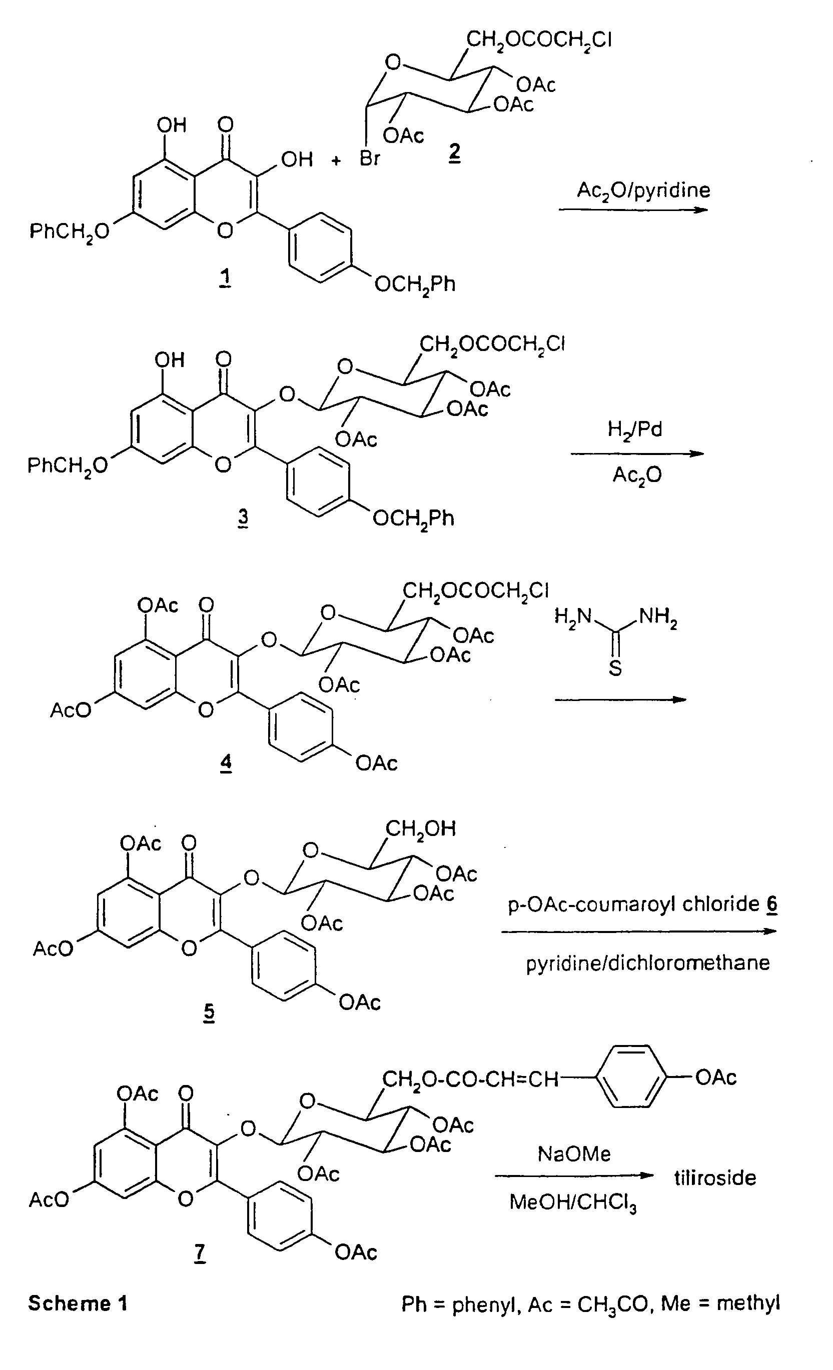

Other compounds of the present invention can be obtained by routine modification of the synthesis shown in scheme 1. Depending on the target molecule, different starting materials are used here, i.e. other optionally protected flavonoids, sugar components and radicals which are to be attached to the sugar component.

The esterification of glycosidic OH groups using aromatic sulfonic acid units can be carried out, for example, by the method described in A.B. Foster et al., J. Chem. Soc. (1954) 3625-3629. After this, the sugar component can, for example, be reacted with a corresponding aromatic sulfonyl chloride in pyridine.

The etherification of glycosidic OH groups using aromatic radicals can be carried out, for example, by the method described in P. Beraud et al., Tetrahedron Let. 30(3) (1989) 325-326. In this Mitsunobu reaction, the etherification is carried out, for example, by dissolving the sugar component in pyridine together with triphenylphosphine PPh₃ and reacting it with a corresponding phenol component and diethyl azodicarboxylate.

The etherification of glycosidic OH groups using radicals of saturated hydrocarbons can be carried out, for example, by the method described in M. Goebel et al., Tetrahedron 53(9) (1997) 3123-3134. The etherification is carried out, for example, by carefully adding sodium hydride to the sugar component in dry dimethylformamide under an inert gas and then carefully reacting the mixture with a suitable alkylating reagent, such as, for example, a corresponding bromide.

The proportion of the compounds of the present invention in the formulation is preferably from 0.001 to 20% by weight, particularly preferably from 0.01 to 10% by weight and especially preferably from 0.05 to 5% by weight, based on the formulation as a whole. The proportion of the compounds of the present invention in the formulation is very especially preferably from 0.05 to 2% by weight, based on the formulation as a whole.

The protective action against oxidative stress or against the effect of free radicals can be further improved if the formulation comprises one or more further antioxidants.

There are many proven substances known from the specialist literature which can be used as antioxidants, for example amino acids (for example glycine, histidine, tyrosine, tryptophan) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example α-carotene, β-carotene, lycopene) and derivatives thereof, chlorogenic acid and derivatives thereof, lipoic acid and derivatives thereof (for example dihydrolipoic acid), aurothioglucose, propylthiouracil and other thiols (for example thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts), and sulfoximine compounds (for example buthionine sulfoximines, homocysteine sulfoximine, buthionine sulfones, penta-, hexa- and heptathionine sulfoximine) in very low tolerated doses (for example pmol to µmol/kg), and also (metal) chelating agents (for example α-hydroxy fatty acids, palmitic acid, phytic acid, lactoferrin), α-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof, vitamin C and derivatives (for example ascorbyl palmitate, magnesium ascorbyl phosphate, ascorbyl acetate), tocopherols and derivatives (for example vitamin E acetate), vitamin A and derivatives (for example vitamin A palmitate), and coniferyl benzoate of benzoin resin, rutinic acid and derivatives thereof, α-glycosyl rutin, ferulic acid, furfurylideneglucitol, carnosine, butylhydroxytoluene, butylhydroxyanisole, nordihydroguaiaretic acid, trihydroxybutyrophenone, quercetin, uric acid and derivatives thereof, mannose and derivatives thereof, zinc and derivatives thereof (for example ZnO, ZnSO₄), selenium and derivatives thereof (for example selenomethionine), stilbenes and derivatives thereof (for example stilbene oxide, trans-stilbene oxide).

Mixtures of antioxidants are likewise suitable for use in the formulations according to the invention. Known and commercial mixtures are, for example, mixtures comprising, as active ingredients, lecithin, L-(+)-ascorbyl palmitate and citric acid (for example Oxynex® AP), natural tocopherols, L-(+)-ascorbyl palmitate, L-(+)-ascorbic acid and citric acid (for example Oxynex® K LIQUID), tocopherol extracts from natural sources, L-(+)-ascorbyl palmitate, L-(+)-ascorbic acid and citric acid (for example Oxynex® L LIQUID), DL-α-tocopherol, L-(+)-ascorbyl palmitate, citric acid and lecithin (for example Oxynex® LM) or butylhydroxytoluene (BHT), L-(+)-ascorbyl palmitate and citric acid (for example Oxynex® 2004).

The proportion of the one or more antioxidants in the formulation is preferably from 0.001 to 5% by weight, particularly preferably from 0.01 to 2% by weight, based on the formulation as a whole.

The protective action of the formulations according to the invention against UV radiation and/or oxidative stress can also be improved if the formulation comprises one or more compounds selected from flavonoids and coumaranones in addition to the compounds of the formula I. Flavonoids are taken to mean the glycosides of flavonones, flavones, 3-hydroxyflavones (= flavonols), aurones, isoflavones and rotenoids [Römpp Chemie Lexikon [Römpp's Lexicon of Chemistry], Volume 9, 1993]. For the purposes of the present invention, however, this term is also taken to mean the aglycones, i.e. the sugar-free constituents, and the derivatives of the flavonoids and aglycones. For the purposes of the present invention, the term flavonoid is furthermore also taken to mean anthocyanidine (cyanidine). For the purposes of the present invention, the term coumaranones is also taken to mean the derivatives thereof.

Preferred flavonoids are derived from flavonones, flavones, 3-hydroxyflavones, aurones and isoflavones, in particular from flavonones, flavones, 3-hydroxyflavones and aurones.

The flavonoids are preferably selected from the following compounds: 4,6,3',4'-tetrahydroxyaurone, quercetin, rutin, isoquercetin, eriodictyol, taxifolin, luteolin, trishydroxyethylquercetin (troxequercetin), trishydroxyethylrutin (troxerutin), trishydroxyethylisoquercetin (troxeisoquercetin), trishydroxyethylluteolin (troxeluteolin) and the sulfates and phosphates thereof. Of the flavonoids, particular preference is given to rutin and troxerutin. Very especial preference is given to troxerutin.

Of the coumaranones, preference is given to 4,6,3',4'-tetrahydroxybenzyl-3-coumaranone.

The proportion of the one or more compounds selected from the flavonoids and coumaranones in the formulation is preferably from 0.001 to 5% by weight, particularly preferably from 0.01 to 2% by weight, based on the formulation as a whole.

The formulations according to the invention may comprise vitamins as further ingredients. The formulations according to the invention preferably comprise vitamins and vitamin derivatives selected from vitamin A, vitamin A propionate, vitamin A palmitate, vitamin A acetate, retinol, vitamin B, thiamine chloride hydrochloride (vitamin B₁), riboflavin (vitamin B₂), nicotinamide, vitamin C (ascorbic acid), vitamin D, ergocalciferol (vitamin D₂), vitamin E, DL-α-tocopherol, tocopherol E acetate, tocopherol hydrogensuccinate, vitamin K₁, esculin (vitamin P active ingredient), thiamine (vitamin B₁), nicotinic acid (niacin), pyridoxine, pyridoxal, pyridoxamine (vitamin B₆), pantothenic acid, biotin, folic acid and cobalamine (vitamin B₁₂), particularly preferably vitamin A palmitate, vitamin C, DL-α-tocopherol, tocopherol E acetate, nicotinic acid, pantothenic acid and biotin.

The formulations according to the invention may furthermore also comprise, as ingredient, ectoin [(S)-1,4,5,6-tetrahydro-2-methyl-4-pyrimidinecarboxylic acid] and then effect protection of skin cells, in particular protection of Langerhans cells. Formulations comprising tiliroside and ectoin are particularly advantageous.

Addition of 1-(2-hydroxyaryl)alkan-1-one oximes (as described, for example, in EP 0 149 242) and preferably of 2-hydroxy-5-methyllaurophenone oxime provides the formulation according to the invention with an advantageous antiinflammatory action. Particularly advantageous are formulations comprising tiliroside and 2-hydroxy-5-methyllaurophenone oxime in which the said substances are present in a weight ratio of from 1 : 10 to 10 : 1. Application forms of formulations of this type are, for example, aftersun preparations.

Preference is furthermore also given to formulations according to the invention which comprise tiliroside and 4,6,3',4'-tetrahydroxybenzyl-3-coumaranone. The said substances are present in these formulations in a weight ratio of from 1 : 10 to 10 : 1.

Further active ingredients can also be incorporated into the formulations according to the invention, for example
- hydroxyectoin [(S,S)-1,4,5,6-tetrahydro-5-hydroxy-2-methyl-4-pyrimidinecarboxylic acid]
- active ingredients which can serve for wound treatment, such as, for example, allantoin
- insect repellents, such as, for example, ethyl 3-[N-n-butyl-N-acetyl]-aminopropionate [CAS No. 52304-36-6]
- sorbitol for skin care [for example Karion® F liquid or Karion® FP liquid]
- biotin
- anti-ageing products, such as, for example, mixtures comprising hydroxyproline or derivatives of hydroxyproline, for example mixtures comprising lecithin, hydroxyproline dipalmitate, sitosterol, linoleic acid, tocopherol, sodium ascorbate, mannitol, phenoxyethanol, methylparaben, ethylparaben, propylparaben, butylparaben, water [for example RonaCare^{™} ASC III® ] or, for example, mixtures comprising lecithin, hydroxylated lecithin, L-hydroxyproline, disodium rutinyl disulfate, phenoxyethanol, mannitol, magnesium ascorbyl phosphate, methylparaben, ethylparaben, propylparaben, butylparaben, sitosterol, tocopherol, sodium ascorbate, water [for example RonaCare^{™} VTA] bisabolol.

The compounds of the formula I can be incorporated into formulations in a conventional manner. Suitable formulations are those for external use, for example as a cream, lotion, gel or as a solution which can be sprayed onto the skin. It is preferred here for the formulation to comprise at least one oil phase and at least one water phase.

Application forms of the formulations according to the invention which may be mentioned are, for example: solutions, emulsions, PIT emulsions, suspensions, pastes, ointments, gels, creams, soaps, surfactant-containing cleansing preparations, lotions, oils, powders, sprays and aerosols. Further application forms are, for example, sticks, shampoos and shower products. In addition to the compounds of the formula I, any desired conventional excipients, adjuvants and optionally further active ingredients may be added to the formulation.

Preferred adjuvants originate from the group consisting of preservatives, antioxidants, stabilisers, solubilisers, vitamins, colorants, odour improvers, film formers, thickeners and humectants.

Solutions and emulsions can comprise the conventional excipients, such as solvents, solubilisers and emulsifiers, for example water, ethanol, isopropanol, ethyl carbonate, ethyl acetate, benzyl alcohol, benzyl benzoate, propylene glycol, 1,3-butyl glycol, oils, in particular cottonseed oil, groundnut oil, maize oil, olive oil, castor oil and sesame oil, glycerol fatty acid esters, polyethylene glycols and fatty acid esters of sorbitan, or mixtures of these substances.

The emulsions can exist in various forms. Thus, they can be, for example, an emulsion or microemulsion of the water-in-oil (W/O) type or of the oil-in-water (O/W) type, or a multiple emulsion, for example of the water-in-oil-in-water (W/O/W) type.

The formulations may also be in the form of emulsifier-free, disperse formulations. They can be, for example, hydrodispersions or Pickering emulsions.

The formulations may also be in the form of PIT emulsions or hydrogels. The formulations may also comprise liposomes, which include, for example, active ingredients.

Suspensions can comprise the conventional excipients, such as liquid diluents, for example water, ethanol or propylene glycol, suspension media, for example ethoxylated isostearyl alcohols, polyoxyethylene sorbitol esters and polyoxyethylene sorbitan esters, microcrystalline cellulose, aluminium metahydroxide, bentonite, agar-agar and tragacanth, or mixtures of these substances.

Pastes, ointments, gels and creams can comprise the conventional excipients, for example animal and vegetable fats, waxes, paraffins, starch, tragacanth, cellulose derivatives, polyethylene glycols, silicones, bentonites, silicic acid, talc and zinc oxide, or mixtures of these substances.

Soaps can comprise the conventional excipients, such as alkali metal salts of fatty acids, salts of fatty acid monoesters, fatty acid protein hydrolysates, isethionates, lanolin, fatty alcohol, vegetable oils, plant extracts, glycerol, sugars, or mixtures of these substances.

Surfactant-containing cleansing products can comprise the conventional excipients, such as salts of fatty alcohol sulfates, fatty alcohol ether sulfates, sulfosuccinic acid monoesters, fatty acid protein hydrolysates, isethionates, imidazolinium derivatives, methyl taurates, sarcosinates, fatty acid amide ether sulfates, alkylamidobetaines, fatty alcohols, fatty acid glycerides, fatty acid diethanolamides, vegetable and synthetic oils, lanolin derivatives, ethoxylated glycerol fatty acid esters, or mixtures of these substances.

Face and body oils can comprise the conventional excipients, such as synthetic oils, such as fatty acid esters, fatty alcohols, silicone oils, natural oils, such as vegetable oils and oily plant extracts, paraffin oils, lanolin oils, or mixtures of these substances.

Powders and sprays can comprise the conventional excipients, for example milk sugar, talc, silicic acid, aluminium hydroxide, calcium silicate and polyamide powder, or mixtures of these substances. Sprays can additionally comprise the conventional propellants, for example chlorofluorocarbons, propane/butane or dimethyl ether.

All compounds or components which can be used in the formulations are either known and commercially available or can be synthesised by known processes.

The formulation can comprise adjuvants which are usually used in formulations of this type, such as, for example, thickeners, plasticisers, humectants, interface-active agents, emulsifiers, preservatives, antifoaming agents, perfumes, waxes, lanolin, propellants, dyes and/or pigments which colour the agent itself or the skin, and other ingredients usually used in cosmetics or dermatology.

The dispersant or solubiliser used can be an oil, wax or other fatty body, a lower monoalcohol or a lower polyol, or mixtures thereof. The particularly preferred monoalcohols or polyols include ethanol, i-propanol, propylene glycol, glycerol and sorbitol.

A preferred embodiment of the invention is an emulsion which is in the form of a protective cream or milk and, in addition to one or more compounds of the formula IA, comprises fatty alcohols, fatty acids, fatty acid esters, in particular triglycerides of fatty acids, lanolin, natural or synthetic oils or waxes and emulsifiers in the presence of water.

Further preferred embodiments are oily lotions based on natural or synthetic oils and waxes, lanolin, fatty acid esters, in particular triglycerides of fatty acids, or oily/alcoholic lotions based on a lower alcohol, such as ethanol, or a glycol, such as propylene glycol, and/or a polyol, such as glycerol, and oils, waxes and fatty acid esters, such as triglycerides of fatty acids. Solid sticks consist of natural or synthetic waxes and oils, fatty alcohols, fatty acids, fatty acid esters, lanolin and other fatty bodies.

If a formulation is in the form of an aerosol, the conventional propellants, such as alkanes, fluoroalkanes and chlorofluoroalkanes, are generally used.

The formulations according to the invention can be prepared with the aid of methods which are well known to the person skilled in the art.

Furthermore, the compounds of the formula IA also have a stabilising action on the formulation. On use in corresponding products, the latter therefore also remain stable for longer and do not change their appearance. In particular, the efficacy of the ingredients, for example vitamins, is also retained on extended use or extended storage.

The compounds of the formula IA can be converted into a suitable dosage form together with at least one solid, liquid and/or semi-liquid excipient or adjuvant and optionally in combination with one or more further active ingredients.

The formulations can be used as medicaments in human or veterinary medicine. Suitable excipients are also organic or inorganic substances which are suitable for enteral (for example oral), parenteral or topical application and do not react with the compounds of the formula IA, for example water, vegetable oils, benzyl alcohols, alkylene glycols, polyethylene glycols, glycerol triacetate, gelatine, carbohydrates, such as lactose or starch, magnesium stearate, talc and Vaseline. Suitable for oral administration are, in particular, tablets, pills, coated tablets, capsules, powders, granules, syrups, juices or drops, suitable for rectal administration are suppositories, suitable for parenteral administration are solutions, preferably oil-based or aqueous solutions, furthermore suspensions, emulsions or implants, and suitable for topical application are ointments, creams or powders. The compounds of the formula IA, may also be lyophilised and the resultant lyophilisates used, for example, for the preparation of injection preparations. The formulations indicated may be sterilised and/or comprise adjuvants, such as lubricants, preservatives, stabilisers and/or wetting agents, emulsifiers, salts for modifying the osmotic pressure, buffer substances, dyes, flavours and/or a plurality of further active ingredients, for example one or more vitamins.

The compounds of the formula IA, are generally preferably administered in doses of between about 1 and 500 mg, in particular between 5 and 100 mg, per dosage unit. The daily dose is preferably between about 0.02 and 10 mg/kg of body weight. However, the specific dose for each patient depends on a very wide variety of factors, for example on the efficacy of the specific compound employed, on the age, body weight, general state of health, sex, on the diet, on the time and method of administration, on the excretion rate, medicament combination and severity of the particular disease to which the therapy applies.

The pharmaceutical formulations comprising one or more compounds of the formula IA, can be prepared with the aid of methods which are well known to the person skilled in the art.

The following examples are intended to illustrate the present invention.

All compounds or components which can be used in the formulations are either known and commercially available or can be synthesised by known methods.

The INCI names of the raw materials used are as follows:

| **Raw material** | **INCI name** |
|---|---|
| Abil WE 09 | Polyglyceryl 4-Isostearate, Cetyl Dimethicone Copolyol, Hexyl Laurate |
| Antaron V-220 | PVP/Eicosene Copolymer |
| Arlacel 80 | Sorbitan Oleate |
| Arlacel 165 V | Glyceryl Stearate, PEG-100 Stearate |
| Avocado oil | Persea Gratissima |
| Beeswax | Beeswax |
| Biobase^{™} EP | Glyceryl Stearate, Cetearyl Alcohol, Sodium Stearoyl Lactylate, Lecithin |
| Carbopol ETD 2050 | Carbomer |
| Cetiol V | Decyl Oleate |
| Cetyl alcohol | Cetyl Alcohol |
| Cetyl isononanoate | Cetyl Isononanoate |
| Cutina HR | Hydrogenated Castor Oil |
| Dimeticon | Dimethicone |
| Eusolex® 232 | Phenylbenzimidazole Sulfonic Acid |
| Eusolex® 2292 | Octyl Methoxycinnamate, BHT |
| Eusolex® 6300 | 4-Methylbenzylidene Camphor |
| Eusolex 8300 | 4-Methylbenzylidene |
| Eusolex® 9020 | Butyl Methoxydibenzoylmethane |
| Eusolex® HMS | Homosalate |
| Eusolex T-Aqua | Aqua (Water), Titanium Dioxide, Alumina, Sodium Metaphosphate, Phenoxyethanol, Sodium Methylparaben |
| Eutanol G | Octyldodecanol |
| Germaben II | Propylene Glycol, Diazolidinyl Urea, Methylparaben, Propylparaben |
| Germaben II-E | Propylene Glycol, Diazolidinyl Urea, Methylparaben, Propylparaben |
| Glycerin | Glycerin |
| Glycerin (87%) | Glycerin |
| Glycerin (87% extra pure) | Glycerin |
| Glycerin, anhydrous | Glycerin |
| Hetester PHA | Propylene Glycol Isoceteth-3 Acetate |
| Hexyl laurate | Hexyl Laurate |
| Imwitor 960 K flakes | Glyceryl Stearate SE |
| Isolan PDI | Diisostearoyl Polyglyceryl 3-Diisostearate |
| Isopropyl myristate | Isopropyl Myristate |
| Isopropyl palmitate | Isopropyl Palmitate |
| Jojoba oil | Buxus Chinensis (Jojoba Oil) |
| Karion F liquid | Sorbitol |
| Keltrol RD | Xanthan Gum |
| Magnesium sulfate | Magnesium Sulfate |
| Magnesium sulfate heptahydrate | Magnesium Sulfate |
| Methyl 4-hydroxybenzoate | Methylparaben |
| Miglyol 812 | Caprylic/Capric Triglyceride |
| Miglyol 812 N | Caprylic/Capric Triglyceride |
| Miglyol 812, neutral oil | Caprylic/Capric Triglyceride |
| Mirasil CM5 | Cyclomethicone |
| Mirasil DM 350 | Dimethicone |
| Montanov 68 | Cetearyl Alcohol, Cetearyl Glucoside |
| Sodium chloride | Sodium Chloride |
| Sodium hydroxide solution, 10% | Sodium Hydroxide |
| Oxynex® K | PEG-8, Tocopherol, Ascorbyl Palmitate, Ascorbic Acid, Citric Acid |
| Panthenol-D | Panthenol |
| Paracera M | Microwax |
| Paraffin oil, liquid | Mineral Oil |
| Perfume oil TND-2417 | Perfume |
| Pemulen TR-1 | Acrylate/C₁₀₋₃₀ Alkyl Acrylate Crosspolymer |
| Pemulen® TR-2 | Acrylate/C₁₀₋₃₀ Alkyl Acrylate Crosspolymer |
| Performa® V 825 | Synthetic Wax |
| Polyglyceryl 2-dipolyhydroxystearate | Polyglyceryl 2-Dipolyhydroxystearate |
| Prisorine 2021 | Isopropyl Isostearate |
| 1,2-Propanediol | Propylene Glycol |
| Propyl4-hydroxybenzoate | Propylparaben |
| Rhodicare S | Xanthan Gum |
| RonaCare^{™} ASC III | Aqua, Lecithin, Dipalmitoyl Hydroxyproline, Phenoxyethanol, Tall Oil Sterol, Linoleic Acid, Tocopherol, Sodium Ascorbate, Mannitol, Methylparaben, Ethylparaben, Propylparaben, Butylparaben |
| RonaCare^{™} Bisabolol | Bisabolol |
| RonaCare^{™} Ectoin | Ectoin |
| RonaCare^{™} LPO | Lauryl p-Cresol Ketoxime |
| RonaCare^{™} Tocopherol acetate | Tocopheryl Acetate |
| Sepigel 305 | Polyacrylamide, C₁₃₋₁₄ Isoparaffin, |
| | Laureth-7 |
| SFE 839 | Cyclopentasiloxane, Dimethicone/ Vinyldimethicone Crosspolymer |
| Shea butter | Shea Butter |
| Steareth-2 | Steareth-2 |
| Steareth-10 | Steareth-10 |
| Stearic acid | Stearic Acid |
| DL-α-tocopherol acetate | Tocopherol Acetate |
| Triethanolamine | Triethanolamine |
| Triethanolamine extra pure | Triethanolamine |
| Water, demineralised | Aqua (Water) |
| Zinc stearate | Zinc Stearate |

### Examples

### Example A

### Preparation of sulfated tiliroside, sodium salt

200 ml of water and 19.4 g of 32% sodium hydroxide solution (155.2 mmol) are added to 29.7 g of tiliroside (50 mmol) with stirring. 19.9 g of pyridine sulfone (125 mmol) are subsequently added, and the pH is adjusted to pH 8 by addition of 32% sodium hydroxide solution. The reaction batch is stirred under N₂ for 12 hours and then filtered, and the filtrate is concentrated to 50 g under reduced pressure (T = 60°C; p = 100 mbar). 250 ml of methanol are added dropwise to the concentrated filtrate over the course of 1 hour, and the precipitated solid (sodium sulfate) is filtered off. Drying gives sulfated tiliroside, sodium salt.

### Example 1

**Lotion (W/O) for application to the skin**

| | | **% by wt.** |
|---|---|---|
| **A** | Polyglyceryl 2-Dipolyhydroxystearate | 5.0 |
| | Beeswax | 0.5 |
| | Zinc Stearate | 0.5 |
| | Hexyl Laurate | 9.0 |
| | Cetyl lsononanoate | 6.0 |
| | Shea Butter | 0.5 |
| | DL-α-Tocopherol Acetate | 1.0 |
| | Tiliroside | 0.5 |
| **B** | Glycerin | 5.0 |
| | Magnesium Sulfate Heptahydrate | 1.0 |
| | Preservative | q.s. |
| | Water, Demineralised | to 100 |

### Preparation

Phase A is heated to 75°C and phase B to 80°C. Phase B is added slowly to phase A with stirring. After homogenisation, the mixture is cooled with stirring. Perfumes are added at a temperature of 40°C.

The preservatives used are the following:
0.05% of propyl 4-hydroxybenzoate
0.15% of methyl 4-hydroxybenzoate

### Example 2

**Lotion (W/O) for application to the skin**

| | | **% by wt.** |
|---|---|---|
| **A** | Polyglyceryl 2-Dipolyhydroxystearate | 5.0 |
| | Beeswax | 0.5 |
| | Zinc Stearate | 0.5 |
| | Hexyl Laurate | 9.0 |
| | Cetyl Isononanoate | 6.0 |
| | Shea Butter | 0.5 |
| | DL-α-Tocopherol Acetate | 1.0 |
| **B** | Sulfated Tiliroside, Sodium Salt (Example A) | 1.0 |
| | Glycerin | 5.0 |
| | Magnesium Sulfate Heptahydrate | 1.0 |
| | Preservative | q.s. |
| | Water, Demineralised | to 100 |

### Preparation

Phase A is heated to 75°C and phase B to 80°C. Phase B is added slowly to phase A with stirring. After homogenisation, the mixture is cooled with stirring. Perfumes are added at a temperature of 40°C.

The preservatives used are the following:
0.05% of propyl 4-hydroxybenzoate
0.15% of methyl 4-hydroxybenzoate

### Example 3

**Lotion (W/O) for application to the skin**

| | | **% by wt.** |
|---|---|---|
| **A** | 4,6,3',4'-Tetrahydroxybenzyl-3-coumaranone | 1.0 |
| | Polyglyceryl 2-Dipolyhydroxystearate | 5.0 |
| | Beeswax | 0.5 |
| | Zinc Stearate | 0.5 |
| | Hexyl Laurate | 9.0 |
| | Cetyl Isononanoate | 6.0 |
| | Shea Butter | 0.5 |
| | DL-α-Tocopherol Acetate | 1.0 |
| | Tiliroside | 1.0 |
| **B** | Glycerin | 5.0 |
| | Magnesium Sulfate Heptahydrate | 1.0 |
| | Preservative | q.s. |
| | Water, Demineralised | to 100 |

### Preparation

Phase A is heated to 75°C and phase B to 80°C. Phase B is added slowly to phase A with stirring. After homogenisation, the mixture is cooled with stirring. Perfumes are added at a temperature of 40°C.

The preservatives used are the following:
0.05% of propyl 4-hydroxybenzoate
0.15% of methyl 4-hydroxybenzoate

### Example 4

A cream (O/W) comprising ectoin is prepared from the following components:

| | | | **% by wt**. |
|---|---|---|---|
| **A** | Paraffin, Liquid | (1) | 8.0 |
| | Isopropyl Myristate | (1) | 4.0 |
| | Mirasil CM5 | (2) | 3.0 |
| | Stearic Acid | (1) | 3.0 |
| | Arlacel 165 V | (3) | 5.0 |
| | Tiliroside | | 1.0 |
| **B** | Glycerin (87%) | (1) | 3.0 |
| | Germaben II | (4) | 0.5 |
| | Water, Demineralised | | to 100 |
| **C** | RonaCare^{™} Ectoin | (1) | 1.0 |

### Preparation

Firstly, phases A and B are heated separately to 75°C. Phase A is then added slowly to phase B with stirring, and stirring is continued until a homogeneous mixture has formed. After homogenisation, the emulsion is cooled to 30°C with stirring. The mixture is subsequently warmed to 35°C, phase C is added, and the mixture is stirred until homogeneous.

### Sources of supply

(1) Merck KGaA
(2) Rhodia
(3) Uniqema
(4) ISP

### Example 5

**Topical composition as W/O emulsion**

| | | | **% by wt.** |
|---|---|---|---|
| **A** | Isolan PDI | (2) | 3.0 |
| | Paraffin Oil, Liquid | (1) | 17.0 |
| | Isopropyl Myristate | | 5.0 |
| | Beeswax | | 0.2 |
| | Cutina HR | (2) | 0.3 |
| | Tiliroside | | 1.0 |
| **B** | Water, Demineralised | | to 100 |
| | Glycerin (87%) | | 4.0 |
| | Magnesium Sulfate | | 1.0 |
| | Germaben II-E | (3) | 1.0 |
| **C** | RonaCare^{™} LPO | (1) | 2.0 |

### Preparation

Phases A and B are heated to 75°C. Phase B is added to phase A with stirring. The mixture is subsequently homogenised for 2 minutes at 9000 rpm using the Turrax. The resultant mixture is cooled to from 30 to 35°C, and C is stirred in.

### Sources of supply

(1) Merck KGaA
(2) Goldschmidt AG
(3) ISP

### Treatment of neurodermatitis

The base cream used for the experiments described below was Nivea^{™} cream (Beiersdorf AG, Hamburg). (Ingredients: Aqua, Paraffinum Liquidum, Cera Microcristallina, Glycerin, Lanolin Alcohol (Eucerit® ), Paraffin, Magnesium Sulfate Decyl Oleate, Octyldodecanol, Aluminum Stearate, Panthenol, Citric Acid, Magnesium Stearate, Perfume.)

### Example 1:

A female test subject (31) has suffered since birth from chronic neurodermatitis, which is being treated with Prednisolon^{™}. In the acute stage, base cream containing 1% of added tiliroside was applied thinly to the diseased skin areas twice daily for three days. All acute symptoms disappeared completely within 2 days, and no new episode occurred within five weeks. Identical observations were made in the case of recurrences and have since lasted without loss of activity.

### Example 2:

A female test subject (42) suffers from neurodermatitis. Treatment with base cream (1% of added tiliroside) showed a significant improvement in the skin picture compared with tiliroside-free base cream. The reddening disappeared completely with tiliroside-containing base cream.

## Claims

1. Use of a compound of the formula IA 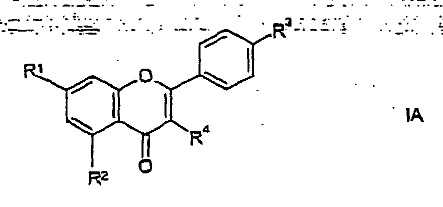 in which
R¹, R² are each, independently of one another, OH, CH₃COO, an alkoxy radical having from 1 to 8 carbon atoms or a monoglycoside radical,
R³ OH
R⁴ is a mono- or diglucoside radical, where at least one group selected from 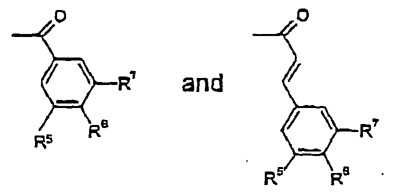 where R⁵, R⁶ and R⁷ may each, independently of one another, be H or have the meaning of the radicals R¹ to R³, is bonded to the glucoside radical, in each case via an -O- group, and in which one or more hydrogen atoms in the OH groups of the glucoside radical(s) may each, independently of one another, also be replaced by acetyl or by alkyl radicals having from 1 to 8 carbon atoms, and where, in each case independently of one another, sulfate or phosphate may also be bonded to one or more hydroxyl groups of the compounds of the formula IA
for the preparation of a formulation further comprising a skin-tolerated excipient, whereby the formulation is suitable for the prophylaxis and/or therapy of atopic eczema.

2. Use according to Claim 1, **characterised in that** the formulation is a formulation which is suitable for the prophylaxis and/or therapy of milk crust, neurodermatitis, prurigo and dermatitis sicca.

3. Use according to Claim 1, **characterised in that** the compounds of the formula IA are selected from the compounds of the formulae IA1 and IA2

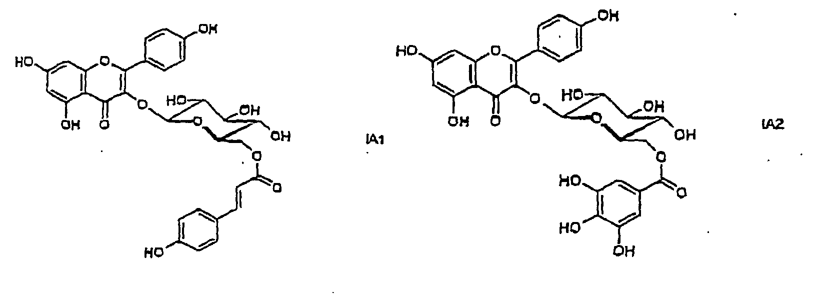

4. Use according to at least one of the preceding claims, **characterised in that** the compound of the formula IA1 is used in the form of a plant extract, a purified plant extract or in the form of the pure substance prepared from the plant extract, where the plant extract preferably comprises from 5 to 90% by weight of the compound of the formula IA1 and has particularly preferably been isolated by extraction from the Sida glaziovii plant.

5. Use according to at least one of the preceding claims, **characterised in that** the one or more compounds of the formula IA are present in the formulation in amounts of from 0.001 to 20% by weight

6. Use according to at least one of the preceding claims, **characterised in that** the formulation comprises one or more antioxidants.

7. Use according to at least one of the preceding claims, **characterised in that** the compound of the formula IA is tiliroside.

8. Use according to claim 7, where tiliroside is in the form of a plant extract, a purified plant extract or in the form of the pure substance prepared from the plant extract.

9. Use according to at least one of the preceding claims, **characterised in that** the formulation further comprises one or more further active ingredients having a inflammation-inhibiting action being selcted from glucocorticoids or tacrolimus.

10. Formulation for topical application comprising
a) at least one compound of the formula IA according to Claim 1,
b) a skin-tolerated excipient,
c) one or more further active ingredients having a inflammation-inhibiting action being selcted from glucocorticoids or tacrolimus.

## Patentansprüche

1. Verwendung einer Verbindung der Formel IA 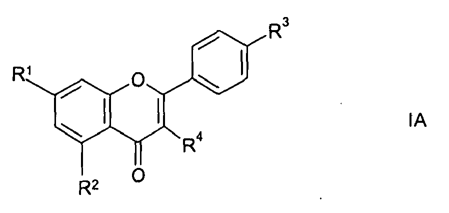 worin
R¹, R² jeweils unabhängig voneinander OH, CH₃COO, einen Alkoxyrest mit 1 bis 8 C-Atomen oder einen Monoglykosidrest bedeuten,
R³ OH bedeutet,
R⁴ ein Mono- oder Diglykosidrest ist, wobei an den Glykosidrest jeweils über eine Gruppe -O-mindestens eine Gruppe ausgewählt aus 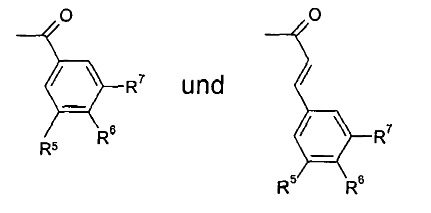 gebunden ist,
wobei R⁵, R⁶ und R⁷ jeweils unabhängig voneinander H sein können oder die Bedeutung der Reste R¹ bis R³ besitzen, und worin ein oder mehrere Wasserstoffatome in den OH-Gruppen des oder der Glykosidreste jeweils unabhängig voneinander auch durch Acetyl oder durch Alkylreste mit 1 bis 8 C-Atomen ersetzt sein können und wobei an eine oder mehrere Hydroxygruppen der Verbindungen der Formel IA jeweils unabhängig voneinander auch Sulfat oder Phosphat gebunden sein kann, zur Herstellung einer Zubereitung weiter enthaltend einen hautverträglichen Träger, wobei die Zubereitung zur Prophylaxe und/oder Therapie von atopischem Ekzem geeignet ist.

2. Verwendung nach Anspruch 1, **dadurch gekennzeichnet, dass** es sich bei der Zubereitung um eine Zubereitung geeignet zur Prophylaxe und/oder Therapie von Milchschorf, Neurodermitis, Prurigo und Dermatitis sicca handelt.

3. Verwendung nach Anspruch 1, **dadurch gekennzeichnet, dass** die Verbindungen der Formel IA ausgewählt sind aus den Verbindungen der Formeln IA1 und IA2

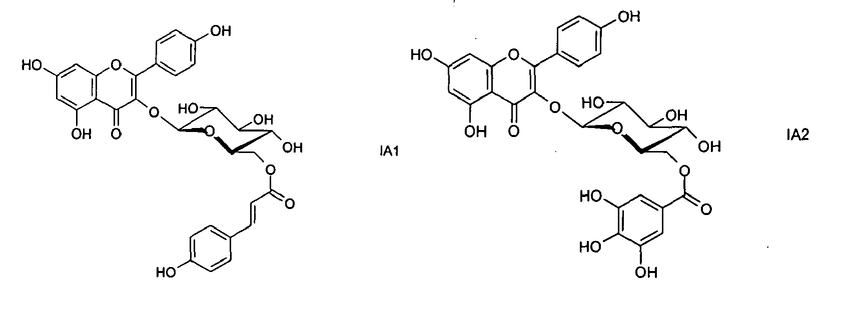

4. Verwendung nach mindestens einem der vorstehenden Ansprüche, **dadurch gekennzeichnet, dass** die Verbindung der Formel IA1 in Form eines Pflanzenextrakts, eines aufgereinigten Pflanzenextrakts oder in Form der aus dem Pflanzenextrakt hergestellten Reinsubstanz verwendet wird, wobei der Pflanzenextrakt vorzugsweise 5 bis 90 Gew.-% der Verbindung der Formel IA1 enthält und insbesondere bevorzugt durch Extraktion aus der Pflanze Sida glaziovii gewonnen worden ist.

5. Verwendung nach mindestens einem der vorstehenden Ansprüche, **dadurch gekennzeichnet, dass** die eine oder die mehreren Verbindungen der Formel IA in Mengen von 0,001 bis 20 Gew.-% in der Zubereitung enthalten sind.

6. Verwendung nach mindestens einem der vorstehenden Ansprüche, **dadurch gekennzeichnet, dass** die Zubereitung ein oder mehrere Antioxidantien enthält.

7. Verwendung nach mindestens einem der vorstehenden Ansprüche, **dadurch gekennzeichnet, dass** die Verbindung der Formel IA Tilirosid ist.

8. Verwendung nach Anspruch 7, wobei Tilirosid in Form eines Pflanzenextrakts, eines aufgereinigten Pflanzenextrakts oder in Form der aus dem Pflanzenextrakt hergestellten Reinsubstanz vorliegt.

9. Verwendung nach mindestens einem der vorstehenden Ansprüche, **dadurch gekennzeichnet, dass** die Zubereitung weiter einen oder mehrere weitere Wirkstoffe mit entzündungshemmender Wirkung, die aus Glukokortikoiden oder Tacrolimus ausgewählt sind, enthält.

10. Zubereitung zur topischen Anwendung enthaltend
a) mindestens eine Verbindung der Formel IA nach Anspruch 1,
b) einen hautverträglichen Träger,
c) einen oder mehrere weitere Wirkstoffe mit entzündungshemmender Wirkung, die aus Glukokortikoiden oder Tacrolimus ausgewählt sind.

## Revendications

1. Utilisation d'un composé de la formule IA 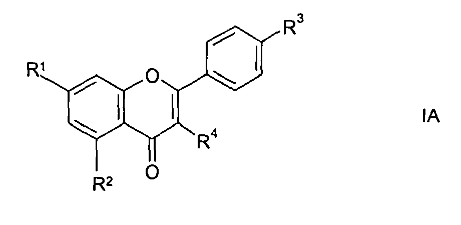 dans laquelle
R¹, R² sont, chacun indépendamment l'un de l'autre, OH, CH₃COO, un radical alkoxy comportant de 1 à 8 atomes de carbone ou un radical monoglycoside,
R³ est OH
R⁴ est un radical mono- ou diglucoside où au moins un groupe, choisi parmi 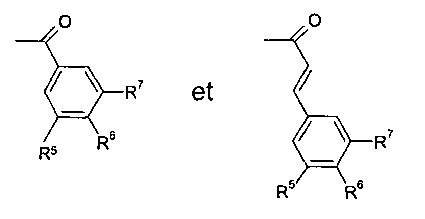 où R⁵, R⁶ et R⁷ peuvent, chacun indépendamment les uns des autres, être H ou présenter la signification des radicaux R¹ à R³, est lié au radical glucoside, dans chaque cas via un groupe -O-, et où un ou plusieurs atomes d'hydrogène dans les groupes OH du radical ou des radicaux glucoside peuvent, chacun indépendamment les uns des autres, être également remplacés par des radicaux acétyle ou alkyle comportant de 1 à 8 atomes de carbone et où, dans chaque cas indépendamment les uns des autres, du sulfate ou du phosphate peut également être lié à un ou plusieurs groupes hydroxyle des composés de la formule IA pour la préparation d'une formulation comprenant en outre un excipient toléré par la peau, d'où il résulte que la formulation convient pour la prophylaxie et/ou la thérapie de l'eczéma atopique.

2. Utilisation selon la revendication 1, **caractérisée en ce que** la formulation est une formulation qui convient pour la prophylaxie et/ou la thérapie de la croûte de lait, de la névrodermatite, du prurigo et de la dermite sèche.

3. Utilisation selon la revendication 1, **caractérisée en ce que** les composés de la formule IA sont choisis parmi les composés des formules IA1 et IA2

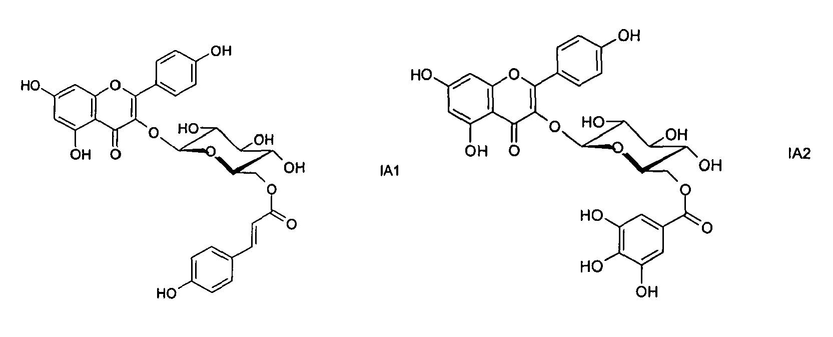

4. Utilisation selon au moins l'une des revendications précédentes, **caractérisée en ce que** le composé de la formule IA1 est utilisé sous la forme d'un extrait de plante, d'un extrait de plante purifié ou sous la forme de la substance pure préparée à partir de l'extrait de plante, où l'extrait de plante comprend de préférence de 5% en poids à 90% en poids du composé de la formule IA1 et a été de façon particulièrement préférable isolé par extraction à partir de la plante Sida glaziovii.

5. Utilisation selon au moins l'une des revendications précédentes, **caractérisée en ce que** les un ou plusieurs composés de la formule IA sont présents dans la formulation selon des quantités qui vont de 0,001 % en poids à 20% en poids.

6. Utilisation selon au moins l'une des revendications précédentes, **caractérisée en ce que** la formulation comprend un ou plusieurs antioxydants.

7. Utilisation selon au moins l'une des revendications précédentes, **caractérisée en ce que** le composé de la formule IA est tiliroside.

8. Utilisation selon la revendication 7, dans laquelle la tiliroside est sous la forme d'un extrait de plante, d'un extrait de plante purifié ou sous la forme de la substance pure préparée à partir de l'extrait de plante.

9. Utilisation selon au moins l'une des revendications précédentes, **caractérisée en ce que** la formulation comprend en outre un ou plusieurs ingrédients actifs supplémentaires présentant une action inhibitrice d'inflammation qui sont choisis parmi les glucocorticoïdes ou les tacrolimus.

10. Formulation pour une application topique comprenant :
a) au moins un composé de la formule IA selon la revendication 1,
b) un excipient toléré par la peau ; et
c) un ou plusieurs ingrédients actifs supplémentaires présentant une action inhibitrice d'inflammation qui sont choisis parmi les glucocorticoïdes ou les tacrolimus.
